# EUROPÄISCHE PATENTSCHRIFT

(11) **EP 0 961 811 B1**
(45) Veröffentlichungstag und Bekanntmachung des Hinweises auf die Patenterteilung: **24.01.2001**
(21) Anmeldenummer: 98912468.0
(22) Anmeldetag: 12.03.1998
(51) Int. Cl.: C08L 83/04, A61K 6/10

(54) **LAGERSTABILE, PERMANENT WASSERBENETZBARE, VULKANISATE ERGEBENDE POLYSILOXANMASSE**
POLYSILOXANE COMPOUND WHICH IS STABLE DURING STORAGE AND PRODUCES VULCANISATES WHICH CAN BE PERMANENTLY WETTED WITH WATER
MATIERE POLYSYLOXANE STABLE AU STOCKAGE ET PRODUISANT DES VULCANISATS HUMECTABLES EN PERMANENCE A L'EAU

(30) Priorität: 18.03.1997 DE 19711314
(43) Veröffentlichungstag der Anmeldung: 08.12.1999
(73) Patentinhaber: Wacker-Chemie GmbH, 81737 München (DE)
(72) Erfinder: STEPP, Michael, D-84489 Burghausen (DE); BINDL, Johann, D-84489 Burghausen (DE); KREIS, Gerhard, D-84489 Burghausen (DE); GARHAMMER, Arnold, D-84359 Simbach (DE)
(74) Vertreter: Fritz, Helmut, Dr.
(86) Internationale Anmeldenummer: EP9801412
(87) Internationale Veröffentlichungsnummer: WO9841579

(56) Entgegenhaltungen:
- EP-A- 0 480 238
- DE-A- 4 010 281
- US-A- 5 580 921

## Beschreibung

Die vorliegende Erfindung betrifft lagerstabile Polysiloxanmassen, die nach dem Vulkanisieren permanent wasserbenetzbare Elastomere ergeben und deren Verwendung als dentale Abformmassen.

Polysiloxanmassen, die zu Elastomeren vulkanisieren, finden breite Anwendung als Abformmassen. Dabei haben sich die additionsvernetzenden Systeme besonders bewährt, da sie schneller abbinden und im Gegensatz zu den kondensationsvernetzenden Systemen keine großen Mengen an problematischem Katalysator benötigen.

Ein wesentlicher Nachteil von Siliconabformpräparaten auf der Basis hydrophober Polysiloxane beruht auf der Tatsache, daß eine präzise Abformung feuchter Oberflächen, wie Gewebe-, Zahn- oder Zahnersatzoberflächen in der Mundhöhle kaum möglich ist, da die Feuchtigkeitsanteile sich tropfenförmig zwischen die abzuformende Oberfläche und die Abformmasse setzen.

Eine oberflächenaktive Abformmasse bewirkt, daß die Feuchtigkeitsanteile auf den abzuformenden Oberflächen zu einem dünnen Feuchtigkeitsfilm spreiten. Dadurch werden Hohlräume im Abdruck vermieden und eine präzise Wiedergabe der Oberflächenstruktur erreicht.

Beispielsweise sind aus der EP-A-480 238 additionsvernetzende wasserbenetzbare Siliconabformmassen bekannt, die speziellen alkoxylierten Fettalkohol oder einen methylierten oder acylierten alkoxylierten Fettalkohol enthalten. Die oberflächenaktiven Agentien werden jedoch durch die schlechte Bindung in den Abformmassen in Kontakt mit wässrigen Medien extrahiert. Dabei geht die Wasserbenetzbarkeit durch beispielsweise Abspülen mit Wasser, Desinfektion, Sterilisation, Erstellen von Duplikaten mit Hilfe wasserhaltiger Abformpräparate, wie Gips verloren, bevor die angestrebten anwendungstechnischen Ziele, wie reproduzierbare Benetzungseigenschaften des Siliconabdrucks nach der Entformung von der feuchten Oberfläche erreicht sind.

In der EP-A-398 745 sind permanent wasserbenetzbare Vulkanisate ergebende Polysiloxanmassen beschrieben, die einvernetzbare hydrophile Modifier enthalten. Die hydrophilen Modifier sind Polysiloxane, die hydrophile Alkylenethergruppen und entweder aliphatische Doppelbindungen oder Si-H Gruppen enthalten. Die in der EP-A-398 745 beschriebenen hydrophilen Modifier weisen herstellungsbedingt einen Gehalt an Edelmetallkatalysator auf. Zusätzlich weisen die bisher herstellbaren Doppelbindungen enthaltenden hydrophilen Modifier geringe Mengen an Si-H Gruppen auf und die bisher herstellbaren Si-H Gruppen enthaltenden hydrophilen Modifier geringe Mengen an Doppelbindungen.

Bei der Verwendung als hydrophile Modifier ergeben die bekannten Mischpolymere aufgrund der vorstehend beschriebenen Mängel weder mit der Zweikomponentenkautschuk-Komponente (A) die eine Polysiloxanmasse mit aliphatischen Doppelbindungen und einen Edelmetallkatalysator umfaßt, noch mit der Komponente (B), die eine Polysiloxanmasse mit Si-H-Gruppen umfaßt, lagerstabile Gemische. In allen Fällen entstehen bei der Lagerung Vulkanisatanteile.

Die getrennte Lagerung des hydrophilen Modifiers ist kein befriedigender Ausweg, da eine Dreikomponentenpolysiloxanmasse bei der Anwendung in der Praxis zu schlecht handhabbar ist.

In der US-A-5,580,921 sind ebenfalls permanent wasserbenetzbare Vulkanisate ergebende Polysiloxanmassen beschrieben, die einvernetzbare Polysiloxane als hydrophilen Modifier aufweisen. Der Modifier wird durch ein sehr aufwendiges Herstellungsverfahren platinfrei erhalten, bei dem hochsiedende Vorstufen des Modifiers destilliert werden. Dadurch ist die Lagerstabilität verbessert.

Der vorliegenden Erfindung liegt die Aufgabe zugrunde, permanent wasserbenetzbare elastomere Vulkanisate ergebende Polysiloxanmassen bereitzustellen, deren Komponenten besonders lagerstabil sind.

Gegenstand der Erfindung sind lagerstabile, permanent wasserbenetzbare elastomere Vulkanisate ergebende Polysiloxanmassen, enthaltend die Bestandteile
1. Organopolysiloxan mit mindestens zwei aliphatischen Doppelbindungen im Molekül,
2. Organopolysiloxan mit mindestens zwei Si-H Gruppen im Molekül,
3. Edelmetallkatalysator und
4. hydrophilen Modifier der allgemeinen Formel I

   [H₂C=CH-A¹-(A²)ₘ-X]ₙB (I),

   in der
   - **A**^{**1**}: einen zweiwertigen C₂-C₁₀-Kohlenwasserstoffrest, der substituiert sein kann mit Halogenatomen,
   - **A**^{**2**}: einen zweiwertigen C₁-C₂₄-Kohlenwasserstoffrest, der durch nicht benachbarte Sauerstoffatome oder Stickstoffatome oder Gruppen der Formeln -NR-, -CO- oder -CO-NR¹-unterbrochen sein kann und zusätzlich noch substituiert sein kann mit Halogenatomen, mit der Maßgabe, daß pro Sauerstoff- oder Stickstoffatom mindestens 5 Kohlenstoffatome vorhanden sind,
   - **X**: eine zweiwertige Gruppe -O-, -CO- oder -COO-,
   - **B**: polare Reste, die Kohlenstoffatome und mindestens 2 nicht benachbarte Sauerstoffatome aufweisen, wobei die Sauerstoffatome als Ethersauerstoff oder in Hydroxylgruppen, C₁-C₄-Acyl- oder C₁-C₃-Trialkylsilylgruppen vorliegen, mit der Maßgabe, daß pro Sauerstoffatom höchstens 3 Kohlenstoffatome vorhanden sind,
   - **m**: die Werte 0 oder 1
   - **n**: ganzzahlige Werte von 1 bis 50,
   - **R** und **R**^{**1**}: jeweils einen einwertigen C₁-C₁₀-Kohlenwasserstoffrest, der substituiert sein kann mit Halogenatomen, bedeuten.

Die lagerbeständige Polysiloxanmasse ist sowohl vor als auch nach dem Vulkanisieren sehr gut wasserbenetzbar. Die Benetzbarkeit der Vulkanisate bleibt auch nach längerem Kontakt mit wässrigen Systemen, beipielsweise nach Sterilisation gut.

Durch Vermischen der Bestandteile 1 bis 4 werden vorzugsweise 2 Komponenten hergestellt, nämlich die den Katalysatorbestandteil 3 enthaltende Komponente A, welche alle Bestandteile, außer Bestandteil 2 enthält und Komponente B, welche alle Bestandteile, außer Bestandteil 3 enthält.

Vorzugsweise enthalten alle Komponenten hydrophilen Modifier. Bei dieser Ausführungsform kann die Gesamtmenge an hydrophilem Modifier in der lagerstabilen Polysiloxanmasse und damit die hydrophilen Eigenschaften beträchtlich erhöht werden, ohne daß Entmischung eintritt. Ein weiterer Vorteil dieser Ausführungsform liegt darin, daß der hydrophile Modifier in beiden Komponenten homogen und in gleicher Konzentration vorhanden sein kann. Dies ist bei der Anwendung moderner Applikationsformen, wie Doppelkammerkartuschen mit aufgesetztem Statikmischer durch die sehr kurzen Mischzeiten von großer Bedeutung.

Der zweiwertige C₂-C₁₀-Kohlenwasserstoffrest **A**^{**1**} hat hydrophoben Charakter. Er kann gesättigt, aliphatisch ungesättigt, aromatisch, linear oder verzweigt sein. Vorzugsweise weist **A**^{**1**} 3 bis 8 Kohlenstoffatome auf.

Der zweiwertige C₁-C₂₄-Kohlenwasserstoffrest **A**^{**2**} hat hydrophoben Charakter. Er kann gesättigt, aliphatisch ungesättigt, aromatisch, linear oder verzweigt sein. Die Sauerstoffatome oder Stickstoffatome oder Gruppen der Formeln -NR-, -CO- oder -CO-NR¹- können Teil der linearen Kette oder von Heterocyclen sein. Vorzugsweise weist A² 5 bis 18 Kohlenstoffatome auf.

Als zweiwertige Gruppe **X** sind -O-, und -COO- bevorzugt, insbesondere -COO-.

Vorzugsweise weist **B** mindestens 2, insbesondere mindestens 3 Sauerstoffatome auf. Vorzugsweise weist **B** höchstens 50, insbesondere höchstens 20 Sauerstoffatome auf.

Der polare Rest **B** ist bevorzugt aufgebaut aus Polyethylenglykoleinheiten [-CH₂CH₂-O-] mit vorzugsweise 1 - 20 Alkylenoxideinheiten, insbesondere mit 3 - 10 Alkylenoxideinheiten oder ein Polyol, mit 3 bis 10 Kohlenstoffatomen, wie Glycerin und Sorbit oder ein Zuckerrest, wie Glucose und Saccharose. **B** kann sowohl freie OH-Gruppen als auch acylierte, silylierte oder alkylierte OH-Gruppen enthalten.

Die acylierten OH-Gruppen sind vorzugsweise mit Ameisen- oder Essigsäure acyliert. Die Trialkylsilylgruppen sind vorzugsweise Trimethylsilylgruppen. Die alkylierten OH-Gruppen sind vorzugsweise Ethoxy- oder insbesondere Methoxygruppen.

**n** gibt die Anzahl der molekular an **B** gebundenen ungesättigten Einheiten [H₂C=CH-A¹-(A²)ₘ-X] an. **n** beträgt vorzugsweise ganzzahlige Werte von 1 bis 10.

**R** und **R**^{**1**} sind vorzugsweise Alkylreste mit 1 bis 6 Kohlenstoffatomen, insbesondere die Methyl-, Ethyl-, n-Propyl und iso-Propylreste.

Bevorzugte Halogenatome als Substituenten von **A**^{**1**}**, A**^{**2**}**, R** und **R**^{**1**} sind Fluor-, Chlor- und Bromatome.

Beispiele für hydrophile Modifier sind:

H₂C=CH-(CH₂)₃-O-(CH₂CH₂O)₈CH₃

H₂C=CH-(CH₂)₄-O-(CH₂CH₂O)₃OCCH₃

H₂C=CH-(CH₂)₉-O-(CH₂CH₂O)₁₁H

H₂C=CH-(CH₂)₉-O-(CH₂CH₂O)₇-CH₃

H₂C=CH-(CH₂)₄-COO-(CH₂CH₂O)₁₄-OC-CH₂CH₃

H₂C=CH-(CH₂)₆-COO-(CH₂CH₂O)₃-CH₃

H₂C=CH-(1,4-cyclohexylen)-O-(CH₂CH₂O)₁₀-CH₃

H₂C=CH-(1,3-cyclohexylen)-O-(CH₂CH₂O)₈-(CH₂)₃-CH=CH₂

H₂C=CH-(1,4-phenylen)-O-(CH₂CH₂O)₆-OSi(CH₃)₃

H₂C=CH-(CH₂)₄-O-(1,3-phenylen)₉-O-(CH₂CH₂O)₁₄-CH₃

H₂C=CH-(CH₂)₃-OOC-(1,4-cyclohexylen)-O-(CH₂CH₂O)₄-CH₃

H₂C=CH-(CH₂)₃-O-(1,4-phenylen)-O-(CH₂CH₂O)₁₀-H

H₂C=CH-(CH₂)₈-CO-(CH₂CH₂O)₁₄-CH₃

H₂C=CH-(CH₂)₈-COO-(CH₂CH₂O)₄-OC-(CH₂)₈-CH=CH₂

H₂C=CH-(CH₂)₈-COO-(CH₂CH₂O)₆Si(CH₃)₃

Sorbitan-mono-10-undecenoat
Sorbitan-mono-10-undecenoat, peracetyliert
Sorbitan-bis-10-undecenoat
Pentaerythritol-mono-10-undecenoat
Pentaerythritol-bis-10-undecenoat, trimethylsilyliert
und Mischungen hieraus.

Besonders bevorzugt als hydrophile Modifier sind die 10-Undecensäureester, bei denen [H₂C=CH-A¹-(A²)ₘ-X] in der allgemeinen Formel I H₂C=CH-(CH₂)₈-COO ist. Diese weisen eine antibakterielle Aktivität auf. Diese Wirkung ist besonders bei der Verwendung im zahntechnischen Bereich von Vorteil, weil das nicht unerhebliche Infektionsrisiko durch den ausgehärteten Formkörper dadurch reduziert werden kann.

Die hydrophilen Modifier sind teilweise kommerziell erhältlich und werden nach allgemein üblichen Methoden der organischen Synthesechemie hergestellt.

Die Polysiloxanmassen enthalten vorzugsweise mindestens 0,1 Gew.-%, insbesondere mindestens 0,5 Gew.-% und vorzugsweise höchstens 5,0 Gew.-%, insbesondere höchstens 10,0 Gew.-% hydrophilen Modifier.

Als Bestandteil 1, nämlich Organopolysiloxan mit mindestens zwei aliphatischen Doppelbindungen im Molekül, handelt es sich vorzugsweise um Organopolysiloxan, das SiC-gebundene C₁-C₆-Alkylreste, insbesondere Methylreste und/oder Phenylreste aufweist und mindestens 2 C₁-C₆-Alkenylreste pro Molekül besitzt, die die aliphatischen Doppelbindungen enthalten. Die bevorzugten Alkenylreste sind Vinylreste und Allylreste. Vorzugsweise enthält ein Molekül nicht mehr als 10 Alkenylreste. Vorzugsweise sind die Organopolysiloxane 1 linear.

Die Viskosität der Organopolysiloxane 1 richtet sich nach gewünschter Viskosität der formulierten Pasten bzw. mechanischem Profil der damit herstellbaren Formkörper und beträgt vorzugsweise 300 bis 200000 mPa.s bei 20°C.

Als Bestandteil 2, nämlich Organopolysiloxan mit mindestens zwei Si-H Gruppen im Molekül, handelt es sich vorzugsweise um Organopolysiloxane, die neben Si-H Gruppen SiC-gebundene C₁-C₆-Alkylreste, insbesondere Methylreste und/oder Phenylreste aufweisen. Vorzugsweise sind die Organopolysiloxane 2 linear. Vorzugsweise enthält ein Molekül nicht mehr als 10, insbesondere nicht mehr als 5 Si-H-Gruppen.

Die Viskosität der Organopolysiloxane 2 beträgt vorzugsweise 20 bis 50000 mPa.s, insbesondere 100 bis 5000 mPa.s bei 20°C.

Die Polysiloxanmassen können vernetzen, wenn mindestens einer der Bestandteile 1,2 oder 4 mindestens 3 vernetzungsfähige Gruppen, nämlich Si-H Gruppen oder aliphatische Doppelbindungen aufweist.

Bestandteil 3, nämlich Edelmetallkatalysator besteht vorzugsweise aus Platinmetallen und/oder deren Verbindungen, insbesondere Platin und/oder dessen Verbindungen. Es können hier alle Katalysatoren eingesetzt werden, die auch bisher zur Addition von Si-H-Gruppen an aliphatisch ungesättigte Verbindungen eingesetzt wurden. Beispiele für solche Katalysatoren sind metallisches und feinverteiltes Platin, das sich auf Trägern, wie Siliciumdioxyd, Aluminiumoxyd oder Aktivkohle befinden kann, Verbindungen oder Komplexe von Platin, wie Platinhalogenide, z. B. PtCl₄, H₂PtCl₆·6H₂O, Na₂PtCl₄·4H₂O, Platin-Olefin-Komplexe, Platin-Alkohol-Komplexe, Platin-Alkoholat-Komplexe, Platin-Ether-Komplexe, Platin-Aldehyd-Komplexe, Platin-Keton-Komplexe, einschließlich Umsetzungsprodukten aus H₂PtCl₆·6H₂O und Cyclohexanon, Platin-Siloxankomplexe, wie Platin-Vinylsiloxankomplexe,insbesondere Platin-Divinyltetramethyldisiloxankomplexe mit oder ohne Gehalt an nachweisbarem anorganisch gebundenem Halogen, Bis-(gamma-picolin)-platindichlorid, Trimethylendipyridinplatindichlorid, Dicyclopentadienplatindichlorid, Dimethylsulfoxydiethylenplatin-(II)-dichlorid sowie Umsetzungsprodukte von Platintetrachlorid mit Olefin und primärem Amin oder sekundärem Amin oder primärem und sekundärem Amin, wie das Umsetzungsprodukt aus in 1-Octen gelöstem Platintetrachlorid mit sec.-Butylamin, oder Ammonium-Platinkomplexe. Besonders bevorzugt sind Platinkomplexe, die aus H₂PtCl₆ hergestellt werden.

Edelmetallkatalysator 3 wird vorzugsweise in Mengen 0,5 bis 500 Gewichts-ppm (Gewichtsteilen je Million Gewichtsteilen), insbesondere 2 bis 400 Gewichts-ppm, jeweils berechnet als elementares Platin und bezogen auf das Gesamtgewicht der in den Bestandteilen 1 und 2 vorliegenden Organopolysiloxane eingesetzt.

Die meisten der vorstehend genannten Platinkatalysatoren sind dermaßen aktiv, daß den Polysiloxanmassen vorzugsweise als Bestandteil 5 ein Inhibitor zugesetzt wird, der nach Vermischen der Komponenten die vorzeitige Vernetzung zum Elastomeren verhindert. Beispiele für Inhibitoren 5 sind acetylenisch ungesättigte Alkohole, wie 3-Methyl-1-butin-3-ol, 1-Ethinylcyclohexan- 1-ol, 3,5-Dimethyl-1-hexin-3-ol und 3-Methyl-1-pentin-3-ol und Inhibitoren auf Vinylsiloxanbasis, wie 1,1,3,3-Tetramethyl-1,3-divinylsiloxan und vinylgruppenhaltige Poly-, Oligo- und Disiloxane.

Die Polysiloxanmassen enthalten vorzugsweise mindestens 0,01 Gew.-%, insbesondere mindestens 0,1 Gew.-% und vorzugsweise höchstens 3,0 Gew.-%, insbesondere höchstens 0,5 Gew.-% Inhibitor 5.

Die Polysiloxanmassen können als Bestandteil 6 Organopolysiloxane enthalten, die weder Si-H Gruppen, noch aliphatische Doppelbindungen aufweisen. Bevorzugt sind bei 20°C flüssige Organopolysiloxane, insbesondere trimethylsilylendgestoppte Polydimethylsiloxane mit einer bevorzugten Viskosität von 50 bis 100000 mPa.s bei 20°C.

Die Polysiloxanmassen können als Bestandteil 7 Füllstoffe enthalten, wie nicht verstärkende Füllstoffe, also Füllstoffe mit einer BET-Oberfläche von bis zu 50 m²/g, wie Quarz, Cristobalit, Diatomeenerde, Calciumsilikat, Zirkoniumsilikat, Montmorillonite, wie Bentonite, Zeolithe, einschließlich der Molekularsiebe, wie Natriumaluminiumsilikat, Metalloxidpulver, wie Aluminium-, oder Zinkoxide bzw. deren Mischoxide, Bariumsulfat, Calciumcarbonat, Gips, Glas- und Kunststoffpulver; verstärkende Füllstoffe, also Füllstoffe mit einer BET-Oberfläche von mehr als 50 m²/g, wie pyrogen hergestellte Kieselsäure, gefällte Kieselsäure und Silicium-Aluminium-Mischoxide großer BET-Oberfläche. Die genannten Füllstoffe können hydrophobiert sein, beispielsweise durch die Behandlung mit Organosilanen bzw. -siloxanen oder durch Verätherung von Hydroxylgruppen zu Alkoxygruppen. Es kann eine Art von Füllstoff, es kann auch ein Gemisch von mindestens zwei Füllstoffen eingesetzt werden. Der Gehalt der Polysiloxanmassen an Bestandteil 7 beträgt vorzugsweise 10 bis 80 Gew.-%.

Die Polysiloxanmassen können als Bestandteil 8 Farbstoffe enthalten. Farbstoffe 8 werden beispielsweise zur Unterscheidung verschiedener Komponenten und zur Mischkontrolle eingesetzt. Beispiele für Farbstoffe 8 sind anorganische und organische Farbpigmente, wie Titandioxid oder Aluminiumspinelle, wie Kobaltaluminiumspinell. Der bevorzugte Gehalt der Polysiloxanmassen an Bestandteil 8 beträgt höchstens 10 Gew.-%.

Die Polysiloxanmassen können als Bestandteil 9 Zusatzstoffe für bestimmte Zwecke enthalten. Geeignete Zusatzstoffe sind Fungizide, Bakterizide, Algicide, Mikrobicide, Geruchsstoffe, Geschmackstoffe, Korrosionsinhibitoren, und, wenn auch nicht bevorzugt, organische Lösungsmittel. Die Polysiloxanmassen enthalten Zusatzstoffe jeweils vorzugsweise in Mengen von 0,001 bis 1 Gew.-%, insbesondere von 0,01 bis 0,1 Gew.-%.

Die Polysiloxanmassen weisen im angemischten pastösen oder fließfähigen Zustand eine gute Affinität zu feuchten Oberflächen, wie der feuchten Zahnhartsubstanz und dem feuchten Zahnfleisch auf und eignen sich deshalb besonders gut als Abformmassen in der Zahnmedizin. Die vernetzten Abformungen können außerdem in der üblichen Zeit von 30 Minuten nach Herausnahme des Abdruckes aus dem Mund des Patienten mit der wäßrigen Gipsaufschlämmung ausgegossen werden ohne daß durch Wasserstoffentwicklung das Gipsmodell verfälscht wird. Sie können auch für Abdrücke anderer Körperteile, wie des Gehörgangs, und für alle Zwecke verwendet werden, für die Siliconelastomere mit hydrophilen Eigenschaften vorteilhaft sind, beispielsweise für Kontaktlinsen, Prothesen oder Implantate und nichtmedizinische Zwecke.

In den nachfolgenden Beispielen sind, falls jeweils nicht anders angegeben,
a) alle Mengenangaben auf das Gewicht bezogen;
b) alle Drücke 0,10 MPa (abs.);
c) alle Temperaturen 20° C;
d) Vinylpolymer 1 bedeutet Polydimethlysiloxan mit α,ω-Vinylgruppen mit einer Viskosität von etwa 7000 mm²/s nach Brookfield bei 25°C;
e) Vernetzer bedeutet wasserfreies Dimethylhydrogensiloxaneinheiten als endständige Einheiten aufweisendes Diorganopolysiloxan aus Dimethylsiloxaneinheiten und Methylhydrogensiloxaneinheiten, worin 10 Dimethylsiloxaneinheiten je Methylhydrogensiloxaneinheit vorliegen, mit einer Viskosität von 150 mPas bei 25°C;
f) Katalysator bedeutet einen Pt-1,3-Di-Vinyl-1,1,3,3-tetramethylsiloxanyl-Komplex;
g) Inhibitor bedeutet ein Dimethylsiloxan mit durchschnittlich 5 bis 10 Dimethylsiloxaneinheiten und α,ω-Vinylgruppen;
h) AZ bedeutet Aushärtezeit;
i) RLF bedeutet relative Luftfeuchtigkeit.

### Beispiele

### Beispiel 1

### Herstellung eines Hydrophilmodifiers der allgemeinen Formel I

50 g 10-Undecensäurechlorid und 25 g Polyethylenglykol 200 (Hoechst AG, Gendorf) wurden bis zur Beendigung der Gasentwicklung bei 80°C gerührt. Nach Zugabe von 1 g Hexamethyldisilazan zur Verringerung des Restsäure- und OH-Gehalts wurde Vakuum angelegt und bei 80°C/4 hPa ausgeheizt. Der Rückstand wurde über 1 g Filterhilfe Seitz Super® filtriert. Man isolierte 55,7 g einer klaren, gelblichen Flüssigkeit, bei der es sich laut ¹H-NMR-Spektrum um den Bis-10-undecensäureester von Polyethylenglykol 200 handelte (Molverhältnis Undecensäurerest : Ethylenglykol = 2 : 4,3).

### Vergleichsbeispiel 1

### Herstellung eines nicht einvernetzbaren, hydrophilen Modifiers analog Beispiel 1

44,5 g Dodecansäurechlorid und 22,2 g Polyethylenglykol 200 (Hoechst AG, Gendorf) wurden bis zur Beendigung der Gasentwicklung bei 80°C gerührt. Nach Zugabe von 1 g Hexamethyldisilazan zur Verringerung des Restsäure- und OH-Gehalts wurde Vakuum angelegt und bei 80°C/4 hPa ausgeheizt. Der Rückstand wurde über 1 g Filterhilfe Seitz Super® filtriert. Man isolierte 47,9 g einer klaren, gelblichen Flüssigkeit, bei der es sich laut ¹H-NMR-Spektrum um den Bis-dodecansäureester von Polyethylenglykol 200 handelte (Molverhältnis Dodecansäurerest : Ethylenglykol = 2 : 4,6).

### Beispiel 2

### Extraktionsversuche an Abmischungen mit dem Modifier aus Beispiel 1, dem nichtvernetzbaren Modifier aus Vergleichsbeispiel 1 und einer Ausgangsmischung

Es wurden drei Mischungen hergestellt:
1. Dentale Siliconabformmasse Komponenten A+B analog US-A-5,580,921, Beispiel 4, jedoch ohne Hydrophilmodifier
2. Dentale Siliconabformmasse von vorstehender Mischung 1 mit zusätzlich 3 Gew.-% erfindungsgemäßem hydrophilen Modifier von Beispiel 1
3. Dentale Siliconabformmasse von Mischung 1 mit zusätzlich 3 Gew.-% nicht erfindungsgemäßem hydrophilem Modifier von Vergleichsbeispiel 1

Jede dieser Mischungen wurde durch Vermischen der Komponenten A und B bei Raumtemperatur ausgehärtet. Nach 4 Tagen Lagerung bei Raumtemperatur wurden die Vulkanisate in einer Soxhlet-Apparatur mit einem Gemisch aus 75 Gew.-% Toluol und 25 Gew.-% 1,2-Dimethoxyethan (Monoglyme) 7 Stunden unter Rückflußbedingungen extrahiert und nach vollständiger Entfernung der Lösungsmittelreste im Vakuum zurückgewogen.

Die nachstehenden Anteile wurden extrahiert:

| | |
|---|---|
| Mischung 1 | 2,7 Gew.-% |
| Mischung 2 | 4,4 Gew.-% |
| Mischung 3 | 5,5 Gew.-% |

Daraus läßt sich berechnen, daß im Gegensatz zu dem Dodecansäurederivat von Vergleichsbeispiel 1, welches zu 93% extrahierbar ist, das erfindungsgemäße 10-Undecensäurederivat von Beispiel 1 bei der Aushärtung zu 44% einvernetzt.

Für die vergleichende Prüfung der Wirkung und der Langzeitstabilität von Dentalmassen mit verschiedenen Hydrophil-Modifiern wurde eine "hydrophobe" Basis-Dentalmasse mit A- und B-Komponente hergestellt (= "Blindwert").

Im Vergleich zum gemessenen Blindwert der hydrophoben Masse wurden auch die Aushärtezeiten bei 37°C der entsprechenden hydrophilen Dentalmassen bestimmt. Hydrophiler Modifier von Beispiel 1 und hydrophiler Modifier analog US-A-5,580,921, Beispiel 1 auf Organosiloxanbasis wurden dabei jeweils in der A-Komponente geprüft. Ein Einsatz des Hydrophil-Modifiers in der A-Komponente ist aufgrund der dann zu erwartenden, höheren Langzeitlagerstabilität der modifierfreien B-Komponente von hohem Interesse.

### Beispiel 3

**a) Herstellung einer Dentalmasse "Lightbody" (A-Komponente)**

| | |
|---|---|
| Vinylpolymer | 56% |
| | |
| Quarzmehl mit einer Korngröße von 1-30µm (Quarzwerke Frechen) | 38% |
| | |
| HDK® H15 (von Wacker-Chemie GmbH) | 4% |
| | |
| Chromoxid Grün Pigment | 0,3% |
| | |
| TiO₂ Pigment | 0,2% |
| | |
| Katalysator | 1,4% |
| | |
| Inhibitor | 0,1% |
| | |
| | 100,0% |
| | |

**b) Herstellung einer Dentalmasse "Lightbody" (B-Komponente)**

| | |
|---|---|
| Vinylpolymer | 41% |
| | |
| Quarzmehl mit einer Korngröße von 1-30µm (Quarzwerke Frechen) | 39% |
| | |
| HDK® H15 von (Wacker-Chemie GmbH) | 4% |
| | |
| Chromoxid Grün Pigment | 1% |
| | |
| Vernetzer | 15% |
| | |
| | 100% |

**c) Einmischung von 4% hydrophilem Modifier von Beispiel 1 in die A-Komponente nach Beispiel 3a)**
**d) Einmischung von 4% hydrophilem Modifier analog US-A-5,580,921 Beispiel 1 in die A-Komponente nach Beispiel 3a)**

Die A-Komponenten nach Beispiel 3c) und 3d) wurden sowohl bei 23°C gelagert, als auch bei 70°C (21 bzw. 42 Tage) einer beschleunigten Hitzealterung unterworfen. Als Blindwert diente die ebenfalls nach diesem Schema "gealterte" hydrophobe Basis-Komponente A gemäß Beispiel 3a.

Zur Bestimmung der jeweiligen Aushärtezeiten bei 37°C wurden sämtliche A-Komponenten mit einer nur bei Raumtemperatur gelagerten B-Komponente im A : B-Mengenverhältnis = 1 : 1 mit einem Cyclovisco-Brabender-Gerät bei 37°C geprüft.

Aushärtezeiten bei 37°C von hydrophilen Dentalmassen A + B (A : B = 1 : 1) gegenüber "Blindwert" (=hydrophobe Masse) in Sekunden:

| Lagerbedingungen 23°C/50%RLF | 4% Modifier von Beispiel 1 AZ 37°C | 4% Modifier analog US-A-5,580,921 AZ 37°C | Blindwert AZ 37°C |
|---|---|---|---|
| 1 Tag 23°C | 235 | 135 | 130 |
| 21 Tage 23°C | 220 | 135 | 130 |
| 42 Tage 23°C | 215 | 140 | 125 |
| 21 Tage 70°C | 200 | 270 | 110 |
| 42 Tage 70°C | 215 | 410 | 90 |

### Ergebnis:

1. Modifier von Beispiel 1 bewirkt in der A-Komponente bei der Hitzealterung während 3 Wochen (= 21 Tage) bzw. 6 Wochen (= 42 Tage) nur eine leichte Abnahme der AZ 37°C. Nach 42 Tagen bei 70°C ist der gemessene Wert mit 215 sec noch immer niedriger als bei der ersten Prüfung nach 1 Tag bei 23°C.
2. Die Masse mit Modifier analog US 5,580,921 in der A-Komponente ist bei 23°C zunächst stabil (AZ 37°C = 135 sec) die Werte nach 70°C Lagerung signalisieren dann aber eine Verdoppelung der AZ 37°C auf 270 bzw. 410 sec.

### Beispiel 4

### Bestimmung des Einvernetzungsgrades verschiedener Hydrophilmodifier in Dentalmassen

Modifier-Extraktion zwecks Bestimmung des Einvernetzungsgrades des neuen Hydrophil-Modifiers von Beispiel 1. Hierzu wurden Vulkanisat-Proben einer Soxhletextraktion analog Beispiel 2 unterworfen. Als Lösungsmittelgemisch wurden 525 ml Toluol mit 175 ml Ethinylglycoldimethylether eingesetzt. Es wurde 5 Stunden extrahiert.

### Proben

1. Blindwert: Dentalmasse "Lightbody-A-Komponente" aus Beispiel 4a, jedoch mit 0,5% Katalysator.
   Dentalmasse "Lightbody-B-Komponente" aus Beispiel 3b jedoch mit 4% Vernetzer mit 0,14 Gew. % H.
2. Dentalmasse "Lightbody-B-Komponente" aus Beispiel 3a, jedoch mit 0,5% Katalysator, 4% hydrophilem Modifier von Beispiel 1.
   Dentalmasse "Lightbody-B-Komponente" aus Beispiel 3b, jedoch mit 4,0% Vernetzer mit 0,14 Gew.% H, 4% hydrophilem Modifier von Beispiel 1.
3. Dentalmasse "Lightbody-A-Komponente" 0,5% Katalysator, +4% hydrophilem Modifier analog US-A-5,580,921
   Dentalmasse "Lightbody-B-Komponente" 4% Vernetzer mit 0,14 Gew.% H, 4% hydrophilem Modifier analog US-A-5,580,921.

| Ergebnis: | | | |
|---|---|---|---|
| | Probe 1 | Probe 2 | Probe 3 |
| Massenverlust | 1,7% | 3,1% | 3,9% |
| Differenz zum | -- | 1,4% | 2,2% |

| Blindwert: | | | |
|---|---|---|---|
| Einvernetzungsgrad Hydrophilmodifier | -- | 65% | 45% |

### Beispiel 5

### Bestimmung der Hydrophilie vor und nach Wasserlagerung der Proben von Beispiel 4

Die Proben wurden 7 Tage in Wasser gelagert, dann 3 Tage bei 23°C getrocknet.

| Kontaktwinkel | Probe 1 | Probe 2 | Probe 3 |
|---|---|---|---|
| (nach Wasserlagerung) | 87° | 60° | 65° |

Ergebnis: Die Hydrophilie ist bei Probe 2 nach Wasserlagerung immer noch gut und bestätigt den hohen Einvernetzungsgrad (= permanente Hydrophilie).

## Patentansprüche

1. Lagerstabile, permanent wasserbenetzbare elastomere Vulkanisate ergebende Polysiloxanmassen, enthaltend die Bestandteile
1. Organopolysiloxan mit mindestens zwei aliphatischen Doppelbindungen im Molekül,
2. Organopolysiloxan mit mindestens zwei Si-H Gruppen im Molekül,
3. Edelmetallkatalysator und
4. hydrophilen Modifier der allgemeinen Formel I
[H₂C=CH-A¹-(A²)ₘ-X]ₙB (I),
in der
**A**^{**1**} einen zweiwertigen C₂-C₁₀-Kohlenwasserstoffrest, der substituiert sein kann mit Halogenatomen,
**A**^{**2**} einen zweiwertigen C₁-C₂₄-Kohlenwasserstoffrest, der durch nicht benachbarte Sauerstoffatome oder Stickstoffatome oder Gruppen der Formeln -NR-, -CO- oder -CO-NR¹-unterbrochen sein kann und zusätzlich noch substituiert sein kann mit Halogenatomen, mit der Maßgabe, daß pro Sauerstoff- oder Stickstoffatom mindestens 5 Kohlenstoffatome vorhanden sind,
**X** eine zweiwertige Gruppe -O-, -CO- oder -COO-,
**B** polare Reste, die Kohlenstoffatome und mindestens 2 nicht benachbarte Sauerstoffatome aufweisen, wobei die Sauerstoffatome als Ethersauerstoff oder in Hydroxylgruppen, C₁-C₄-Acyl- oder C₁-C₃-Trialkylsilylgruppen vorliegen, mit der Maßgabe, daß pro Sauerstoffatom höchstens 3 Kohlenstoffatome vorhanden sind,
**m** die Werte 0 oder 1
**n** ganzzahlige Werte von 1 bis 50,
**R** und **R**^{**1**} jeweils einen einwertigen C₁-C₁₀-Kohlenwasserstoffrest, der substituiert sein kann mit Halogenatomen, bedeuten.

2. Polysiloxanmassen nach Anspruch 1 in Form von 2 Komponenten, nämlich die den Katalysatorbestandteil 3 enthaltende Komponente A, welche alle Bestandteile, außer Bestandteil 2 enthält und Komponente B, welche alle Bestandteile, außer Bestandteil 3 enthält.

3. Polysiloxanmassen nach Anspruch 1 oder 2, bei denen der polare Rest **B** in Bestandteil 4 aus Polyethylenglykoleinheiten [-CH₂CH₂-O-] aufgebaut ist oder ein Polyol mit 3 bis 10 Kohlenstoffatomen oder ein Zuckerrest ist.

4. Polysiloxanmassen nach einem der Ansprüche 1 bis 3, bei denen in Bestandteil 4 der Ausdruck [H₂C=CH-A¹-(A²)ₘ-X] in der allgemeinen Formel I H₂C=CH-(CH₂)₈-COO ist.

5. Polysiloxanmassen nach einem der Ansprüche 1 bis 4, die als Bestandteil 5 einen Inhibitor enthalten.

6. Polysiloxanmassen nach einem der Ansprüche 1 bis 5, die als Bestandteil 6 Organopolysiloxane enthalten, die weder Si-H Gruppen, noch aliphatische Doppelbindungen aufweisen.

7. Polysiloxanmassen nach einem der Ansprüche 1 bis 6, die als Bestandteil 7 Füllstoffe enthalten.

8. Verwendung der Polysiloxanmassen nach einem der Ansprüche 1 bis 7 als dentale Abformmassen.

## Claims

1. Polysiloxanes materials having a long shelf life, giving permanently water-wettable elastomeric vulcanized products and containing the constituents
1. organopolysiloxane having at least two aliphatic double bonds in the molecule,
2. organopolysiloxane having at least two Si-H groups in the molecule,
3. noble metal catalyst and
4. hydrophilic modifier of the general formula I
[H₂C=CH-A¹-(A²)ₘ-X]ₙB (I),
in which
**A**^{**1**} denotes a divalent C₂-C₁₀-hydrocarbon radical which may be substituted by halogen atoms,
**A**^{**2**} denotes a divalent C₁-C₂₄-hydrocarbon radical which may be interrupted by non-neighbouring oxygen atoms or nitrogen atoms or the groups of the formulae -NR-, -CO- or -CO-NR¹- and may additionally be substituted by halogen atoms, with the proviso that at least 5 carbon atoms are present per oxygen or nitrogen atom,
**X** denotes a divalent group -O-, -CO- or -COO-,
**B** denotes polar radicals which have carbon atoms and at least 2 non-neighbouring oxygen atoms, the oxygen atoms being present as ether oxygen or in hydroxyl groups, C₁-C₄-acyl groups or C₁-C₃-trialkylsilyl groups, with the proviso that not more than 3 carbon atoms are present per oxygen atom,
**m** denotes the values 0 or 1,
**n** denotes integral values from 1 to 50,
**R** and **R**^{**1**} each denote a monovalent C₁-C₁₀-hydrocarbon radical which may be substituted by halogen atoms.

2. Polysiloxane materials according to Claim 1, in the form of 2 components, namely the component A, which contains the catalyst constituent 3 and all constituents except for constituent 2, and component B which contains all constituents except for constituent 3.

3. Polysiloxane materials according to Claim 1 or 2, in which the polar radical **B** in constituent 4 is composed of polyethylene glycol units [-CH₂CH₂-O-] or is a polyol having 3 to 10 carbon atoms or a sugar radical.

4. Polysiloxane materials according to any of Claims 1 to 3, in which, in constituent 4, the expression [H₂C=CH-A¹-(A²)ₘ-X] in the general formula I is H₂C=CH-(CH₂)₈-COO.

5. Polysiloxane materials according to any of Claims 1 to 4, which contains an inhibitor as constituent 5.

6. Polysiloxane materials according to any of Claims 1 to 5, which contains, as constituent 6, organopolysiloxanes which have neither Si-H groups nor aliphatic double bonds.

7. Polysiloxane materials according to any of Claims 1 to 6, which contains fillers as constituent 7.

8. Use of the polysiloxane materials according to any of Claims 1 to 7 as dental impression compounds.

## Revendications

1. Compositions de polysiloxanes stables au stockage donnant lieu à des vulcanisats élastomères mouillables à l'eau de manière permanente, comprenant les constituants
1. un organopolysiloxane ayant au moins deux doubles liaisons aliphatiques dans la molécule,
2. un organopolysiloxane ayant au moins deux groupes Si-H dans la molécule,
3. un catalyseur à métal noble et
4. des agents modificateurs hydrophiles de formule générale I
[H₂C=CH-A¹-(A²)ₘ-X]ₙB (I)
dans laquelle
A¹ représente un radical hydrocarboné en C₂-C₁₀ bivalent qui peut être substitué par des atomes d'halogène,
A² représente un radical hydrocarboné en C₁-C₂₄ bivalent qui peut être interrompu par des atomes d'oxygène ou des atomes d'azote non-adjacents ou des groupes de formules -NR-, -CO- ou -CO-NR¹- et peut être en outre également substitué par des atomes d'halogène, à condition qu'au moins 5 atomes de carbone soient présents par atome d'oxygène ou d'azote,
X représente un groupe bivalent -O-, -CO- ou -COO-,
B représente des radicaux polaires renfermant des atomes de carbone et au moins 2 atomes d'oxygène non-adjacents, les atomes d'oxygène étant présents en tant qu'oxygène d'éther ou dans des groupes hydroxy, des groupes C₁-C₄-acyl- ou C₁-C₃-trialkylsilyle, à condition qu'au plus 3 atomes de carbone soient présents par atome d'oxygène,
m vaut 0 ou 1,
n a des valeurs entières de 1 à 50,
R et R¹ représentent chacun un radical hydrocarboné en C₁-C₁₀ monovalent qui peut être substitué par des atomes d'halogène.

2. Compositions de polysiloxanes selon la revendication 1, sous forme de 2 composants, à savoir le composant A comprenant le constituant catalyseur 3 et comprenant tous les constituants, à l'exception du constituant 3, et le composant B comprenant tous les constituants, à l'exception du constituant 3.

3. Compositions de polysiloxanes selon la revendication 1 ou 2, dans lesquelles le radical polaire B dans le constituant 4 est constitué de motifs polyéthylèneglycol [-CH₂CH₂-O-] ou est un polyol ayant de 3 à 10 atomes de carbone ou un radical glucidique.

4. Compositions de polysiloxanes selon l'une quelconque des revendications 1 à 3, dans lesquelles, dans le constituant 4, l'expression [H₂C=CH-A¹-(A²)ₘ-X] dans la formule générale I est H₂C=CH-(CH₂)₈-COO.

5. Compositions de polysiloxanes selon l'une quelconque des revendications 1 à 4, comprenant un inhibiteur en tant que constituant 5.

6. Compositions de polysiloxanes selon l'une quelconque des revendications 1 à 5, comprenant des organopolysiloxanes en tant que constituant 6, lesquels ne renferment ni de groupes Si-H ni de doubles liaisons aliphatiques.

7. Compositions de polysiloxanes selon l'une quelconque des revendications 1 à 6, comprenant des charges en tant que constituant 7.

8. Utilisation des compositions de polysiloxanes selon l'une quelconque des revendications 1 à 7 en tant que compositions d'empreintes dentaires.
